# EUROPÄISCHE PATENTSCHRIFT

(11) **EP 1 789 192 B1**
(45) Veröffentlichungstag und Bekanntmachung des Hinweises auf die Patenterteilung: **16.09.2009**
(21) Anmeldenummer: 05756366.0
(22) Anmeldetag: 05.07.2005
(51) Int. Cl.: B01L 3/00, A61B 5/00, A61B 5/145, G01N 33/543, G01N 33/487

(54) **ANALYTISCHES TESTELEMENT**
ANALYTICAL TEST ELEMENT
ELEMENT DE TEST ANALYTIQUE

(30) Priorität: 09.07.2004 DE 102004033317
(43) Veröffentlichungstag der Anmeldung: 30.05.2007
(73) Patentinhaber: Roche Diagnostics GmbH, 68305 Mannheim (DE); F.HOFFMANN-LA ROCHE AG, 4070 Basel (CH)
(72) Erfinder: KRÄMER, Peter, 67146 Deidesheim (DE)
(74) Vertreter: Dick, Alexander
(86) Internationale Anmeldenummer: PCT/EP2005/007251
(87) Internationale Veröffentlichungsnummer: WO 2006/005483

(56) Entgegenhaltungen:
- EP-A- 0 503 379
- EP-A- 0 732 590
- EP-A- 0 924 520
- EP-A- 1 486 766
- WO-A-01/68238
- WO-A-01/89691
- WO-A-99/29429
- WO-A-03/020134
- WO-A-20/04091403
- DE-A1- 10 220 296
- US-A- 5 636 640

## Beschreibung

Die vorliegende Erfindung betrifft ein analytisches Testelement, ein Testelementmagazin mit einer Vielzahl von analytischen Testelementen und ein System zur Analyse flüssiger Proben mit mindestens einem analytischen Testelement.

Zur Analyse von Proben, beispielsweise Körperflüssigkeiten wie Blut oder Urin, werden häufig Testelement-Analysesysteme verwendet, bei denen die zu analysierenden Proben auf ein Testelement gegeben werden und gegebenenfalls mit einer oder mehreren Reagenzien in einem Testfeld auf dem Testelement reagieren, bevor sie analysiert werden. Die optische, insbesondere photometrische Auswertung von Testelementen stellt eines der gebräuchlichsten Verfahren zur schnellen Bestimmung der Konzentration von Analyten in Proben dar. Photometrische Auswertungen werden allgemein im Bereich der Analytik, der Umweltanalytik und vor allem im Bereich der medizinischen Diagnostik eingesetzt. Insbesondere im Bereich der Blutglukosediagnostik aus Kapillarblut besitzen Testelemente, die photometrisch ausgewertet werden, einen großen Stellenwert.

In den letzten Jahren haben tragbare Messgeräte zur Blutzuckerbestimmung an Bedeutung gewonnen. Sie ermöglichen zu jeder Zeit mittels eines einfach zu bedienenden Messgerätes, einer hinsichtlich des Einstechschmerzes optimierten Stechhilfe und eines Testelements für den einmaligen Gebrauch, Blutzuckermesswerte zu bestimmen und damit eine genauere Insulindosierung des Patienten zur Stabilisierung seines Blutzuckerwertes vorzunehmen. Die Mehrzahl derzeit gebräuchlicher Blutzuckermessgeräte sieht getrennte Einzel-Testelemente, Messgeräte und Stechhilfen vor. Die Einzel-Testelemente werden hierbei von dem Patienten aus einer feuchtigkeitsgeschützten Einzelverpackung entnommen. Blut wird durch Einstich mit einer Stechhilfe gewonnen. Hierauf wird eine erforderliche Mindestmenge an Blut auf das Testelement aufgebracht und eine Messung mit Hilfe des Messgerätes durchgeführt.

Es gibt verschiedene Formen von Testelementen. Bekannt sind zum Beispiel im Wesentlichen quadratische Blättchen, die auch als Slides bezeichnet werden, in deren Mitte sich ein mehrschichtiges Testfeld befindet. Diagnostische Testelemente, die streifenförmig ausgebildet sind, werden als Teststreifen bezeichnet. Zur räumlichen Trennung von Detektionszone und Probenaufgabestelle eines Testelements sind im Stand der Technik kapillare Testelemente bekannt.

In EP 0 924 520 A2 wird ein Sensor und ein Set von Sensoren offenbart. Der Sensor umfasst ein Analyseteil und eine Passage mit zwei Enden. Ein Ende der Passage ist verbunden mit dem Analyseteil. Das andere Ende der Passage ist vor Verwendung geschlossen, so dass das Innere der Passage und der Analyseteil versiegelt sind, um einen Kontakt mit der Umgebung zu verhindern. Wenn der Sensor verwendet werden soll, so wird das andere Ende der Passage geöffnet, um einen Einlass für eine Probe bereitzustellen.

WO 99/29429 betrifft ein analytisches Testelement zur Bestimmung eines Analyten in einer Flüssigkeit, mit einem inerten Träger, einem Nachweiselement und einem zum kapillaren Flüssigkeitstransport befähigten Kanal, der eine Probenaufgabeöffnung an einem und eine Entlüftungsöffnung am anderen Ende des zum kapillaren Flüssigkeitstransport befähigten Kanals besitzt. Der zum kapillaren Flüssigkeitstransport befähigte Kanal wird zumindest teilweise vom Träger und dem Nachweiselement gebildet und reicht in Richtung des kapillaren Transports von der Probenaufgabeöffnung zumindest bis zu der der Entlüftungsöffnung nächstgelegenen Kante des Nachweiselements.

Die Verpackung des jeweiligen Testelements ist so konzipiert, dass sie die wesentlichen Aufgaben zum Erhalt der Funktion der chemischen und biochemischen Bestandteile auf dem Testelement während einer längeren Lagerzeit erfüllt. Diese Aufgaben sind vor allem Schutz vor der Einwirkung von Lichtstrahlen, Schutz vor dem Zutritt von Feuchtigkeit, Schmutz, Keimen und Staub, sowie Schutz vor mechanischer Beeinträchtigung der Testelemente.

Alternativ zu Einzelverpackungen sind Vorratsbehälter bekannt, welche eine Vielzahl von einzeln entnehmbaren Testelementen beinhalten und einen ausreichend großen Trockenmittelvorrat vorsehen, um die durch Öffnen und Entnahme eines Testelements eingeführte Feuchte zu absorbieren und somit eine ausreichende Lagerungszeit für alle im Behälter vorgesehenen Testelemente zu gewährleisten. Ein solcher Vorratsbehälter ist aus EP 0 640 393 B1 bekannt. In dem Vorratsbehältnis stecken die Testelemente wie in einem Köcher, aus dem sie bei geöffnetem Bevorratungssystem entnommen werden können.

Eine weitere anzutreffende Form eines Vorratsbehälters für Testelemente sind Aluminium- oder Kunststoffröhren, die von einem aufzudrückenden oder aufzuschraubenden Stopfen verschlossen sind. Diese Vorratsbehälter haben den Nachteil, dass die einzelnen Testelemente umständlich manuell entnommen werden müssen. Ein Patient, der zum Beispiel einen Blutzuckertest durchführen möchte, muss neben dem Messgerät eine Stechhilfe und einen getrennten Testelement-Vorratsbehälter mit sich führen. Neben dieser Unbequemlichkeit ist es vor allem nachteilig, dass bei Entnahme eines Testelements dieses und/oder ein anderes verunreinigt wird und die Verunreinigung zu falschen Messresultaten führen kann. Es besteht die Gefahr der Kontamination von Teststreifen durch an den Händen des Patienten haftenden Schmutz oder aufgrund des Herausfallens des Testelements.

Als Alternative ist die Lagerung einer bestimmten Anzahl an Testelementen im Messgerät selbst bekannt.

DE 198 19 407 offenbart einen Behälter für Blutzuckermessgeräte oder andere Messgeräte, welche mit Einweg-Teststreifen arbeiten, die zur Messung einem Sensor zuführbar sind, wobei der Behälter aus zwei Teilen besteht, in dessen erstem die Teststreifen magaziniert sind und in dessen zweitem die verbrauchten Teststreifen gesammelt werden. Dabei können die Teststreifen so aneinander gereiht sein, dass sie ein Band bilden, welches ähnlich dem Band in einer Tonbandkassette gespult werden kann. Sie können statt dessen auch so angeordnet sein, dass sie eine runde Scheibe bilden, auf der sie in einem definierten Abstand zueinander im Bereich des Scheibenumfangs angeordnet sind, so dass durch Drehen der Scheibe ein neues Testfeld in die entsprechende Messposition kommt. Eine weitere Möglichkeit ist, dass die Teststreifen einen Stapel bilden, welcher durch eine Mechanik einzeln abgearbeitet wird und die Teststreifen nacheinander in die entsprechende Messposition und nach erfolgter Messung in ein Sammelfach bringt.

Aus EP 0 622 119 A1 sind im Wesentlichen rechteckige Magazine mit zueinander parallelen, in einer Linie nebeneinander liegenden Kammern bekannt

Bei der Lagerung der Testelemente im Messgerät selbst wird durch einen Öffnungsmechanismus des Gerätes der Vorratsbehälter beziehungsweise die Kammer, in der sich ein Testelement befindet, geöffnet und das Testelement durch einen Bewegungsmechanismus in eine Probenaufnahmeposition gebracht

In DE 198 54 316 A1 wird ein Vorratsbehältnis mit separaten wasserdampfdichten Kammern für Testelemente beschrieben. Jede der Kammern weist zumindest zwei gegenüberliegende, durch jeweils eine Siegelfolie verschlossene Öffnungen auf. Zur Entnahme der Testelemente erfolgt ein Herausschieben eines Testelementes aus seiner Kammer mit Hilfe eines Stößels. Der Stößel durchtrennt dabei die Siegelfolie auf der einen Seite der Kammer und drückt dann auf das Testelement, das aufgrund dieses Drucks des Stößels die Siegelfolie auf der gegenüberliegenden Seite durchtrennt, so dass das Testelement aus der Kammer herausgeschoben werden kann. Weitere Mechanismen zur automatischen Testelement-Entnahme aus einem Vorratsbehältnis offenbaren EP 0 738 666 B1 für einen Vorratsbehälter in Form einer Trommel, EP 0 662 626 B1 und EP 0 732 590 A2 für einen Vorratsbehälter in Form einer Diskette und WO 02/08753 A2 für eine gestapelte Lagerung der Testelemente.

Nach der Aufnahme einer Probe (zum Beispiel Blut) auf das entnommene Testelement erfolgt die Detektion und Messdatenauswertung in dem Messgerät Nach der Entfernung des Testelements durch den Patienten oder einen weiteren, in das Gerät integrierten Mechanismus wird der Vorratsbehälter automatisch so positioniert, dass eine weitere Messung erfolgen kann.

Der Schutz der Testelemente, insbesondere vor Eintreten der Feuchtigkeit, erfolgt im Stand der Technik üblicherweise durch Versiegelung des Vorratsbehälters beziehungsweise einzelner Kammern des Vorratsbehälters für die Testelemente. Die Versiegelung ist dabei zum Beispiel in Form eines geeigneten Folienmaterials geringer Wasserdampfdurchlässigkeit ausgeführt (beispielsweise mit adhäsivem Kunststoff beschichtete Aluminiumfolie). Ferner werden die Testelemente häufig durch Einbringen von Trockenmitteln in den Vorratsbehälter beziehungsweise in die Kammern vor Feuchtigkeit geschützt.

Die im Stand der Technik bekannten Vorrichtungen, die die Bereitstellung der Testelemente, die Probenaufnahme- und die Messfunktion in dem Messgerät integrieren, haben den Nachteil, dass sie sehr komplex aufgebaut sind. Diese Komplexität ergibt sich insbesondere daraus, dass jeweils ein Testelement innerhalb der Vorrichtung zu verschiedenen Positionen transportiert werden muss (aus dem Vorratsbehälter zum Probenaufnahmeort, zur Messposition und dann zum Entsorgungsort). Ferner müssen die Testelemente im Stand der Technik aufwendig, insbesondere gegen Feuchtigkeit geschützt werden. Das Befüllen und Versiegeln des in die Vorrichtung integrierten, zur Lagerung der Testelemente dienenden Vorratsbehälters ist ein kosten- und zeitintensives Verfahren.

DE 100 57 832 C1 beschreibt ein Blutanalysegerät mit einem vereinfachten Aufbau, das ein Komplettsystem aus einem Stechelement, einer Blutentnahmevorrichtung, ein Testfeld umfassenden Testelementen und einer Auswerteeinrichtung bildet. Die Testelemente sind in ein Magazin eingesetzt und zur Durchführung mehrerer Messungen nacheinander in eine Arbeitsposition bringbar. Bei Positionierung eines jeweiligen Testelementes in der Arbeitsposition ist ein Stechelement durch das Testelement hindurchstossbar und in die Hautoberfläche eines Benutzers einstechbar. Das aus der Hautoberfläche austretende Blut beaufschlagt direkt das Testelement. Dieses Blutanalysegerät hat den Nachteil, dass das Testfeld selbst durchstochen wird, so dass toxische Bestandteile des Testfeldes an der Stechhilfe haften bleiben können und in die erzeugte Stechwunde in der Haut des Benutzers eingetragen werden können.

Aufgabe der vorliegenden Erfindung ist es daher, ein analytisches Testelement, ein Testelement-Magazin und ein System zur Analyse flüssiger Proben mit mindestens einem analytischen Testelement bereitzustellen, die die oben genannten Nachteile des Standes der Technik vermeiden. Insbesondere soll der Eintritt von Feuchtigkeit in die Testelemente vermieden werden. Dabei soll außerdem die Herstellung der Testelemente, des Testelement-Magazins und des Analysesystems vereinfacht und deren Komplexität verringert werden.

Diese Aufgabe wird erfindungsgemäß gelöst durch ein analytisches Testelement zur Analyse einer flüssigen Probe, enthaltend einen zum kapillaren Transport der flüssigen Probe geeigneten Kanal mit einer Eintrittsöffnung für die flüssige Probe und einer Entlüftungsöffnung, wobei in dem Kanal, beabstandet zu der Eintrittsöffnung, mindestens ein Testfeld angeordnet ist, wobei das Testelement einen mit einer Versiegelung verschlossenen Probenaufnahmeort umfasst, der so ausgelegt ist, dass durch ein Öffnen der Versiegelung durch Durchstoßen oder Aufschneiden gleichzeitig der Probenaufnahmeort und die Eintrittsöffnung des Kanals zur Umgebung des Testelements hin öffenbar ist und die flüssige Probe durch das Testelement über den Probenaufnahmeort und die Eintrittsöffnung in den Kanal zur Analyse in dem Testfeld aufnehmbar ist.

Das erfindungsgemäße Testelement hat den Vorteil, dass das Testelement selbst weitgehend wasserdampfundurchlässig und schmutzabweisend durch die Versiegelung verschlossen ist und somit für den Zeitraum der Benutzung im Gerät keine getrennte Aufbewahrungsmöglichkeit zum Schutz vor eintretender Feuchtigkeit oder Verunreinigung benötigt. Der eine Messung vorbereitende Schritt des Auspackens eines Testelements kann somit entfallen, wodurch sich eine vereinfachte Handhabung für den Benutzer beziehungsweise ein einfacherer Aufbau eines Messgeräts, in das die Testelemente bereits integriert sind, ergibt. Durch das Öffnen der Versiegelung am Probenaufnahmeort werden auch die Eintrittsöffnung des Kanals geöffnet, so dass das Testelement mit einem einzigen Öffnungsvorgang einsatzbereit ist. Der zum kapillaren Transport der flüssigen Probe geeignete Kanal mit integriertem Testfeld hat den Vorteil, dass durch die Kapillarkräfte ein automatischer Transport der flüssigen Probe zum Testelement, das den Detektionsbereich darstellt, erfolgt. Die zu analysierende Probe kann direkt auf den Probenaufnahmeort gegeben werden.

Die Entlüftungsöffnung stellt eine Entlüftung des Kanals bei dem kapillaren Transport der flüssigen Probe über die Eintrittsöffnung in dem Kanal zum Testfeld sicher. Sie kann bei dem erfindungsgemäßen Testelement bereits vor dem Öffnen der Versiegelung zur Umgebung des Testelements offen sein. Dabei kann eine Kontamination des Testfeldes über die Entlüftungsöffnung durch eine geeignete Führung des Kanals vermieden werden. In einer anderen Ausführungsform der vorliegenden Erfindung mündet die Entlüftungsöffnung in einen ausreichend großen, zur Umgebung des Testelements hin abgeschlossenen Hohlraum, der eine Entlüftung des Kanals über die Entlüftungsöffnung ohne eine Öffnung des Hohlraums zur Umgebung hin erlaubt. Es ist ferner möglich, dass die Entlüftungsöffnung separat versiegelt ist und durch ein Öffnen dieser separaten Versiegelung zur Umgebung des Testelements hin geöffnet wird. In einer bevorzugten Ausführungsform der vorliegenden Erfindung werden durch ein Öffnen nur einer Versiegelung gleichzeitig der Probenaufnahmeort, die Eintrittsöffnung des Kanals und die Entlüftungsöffnung des Kanals zur Umgebung des Testelements hin geöffnet

Das Testfeld ist in dem Kanal vorzugsweise zwischen der Eintrittsöffnung und der Entlüftungsöffnung angeordnet. In einer bevorzugten Ausführungsform der vorliegenden Erfindung ist die Eintrittsöffnung an einem Ende des Kanals und die Entlüftungsöffnung am anderen Ende des Kanals angeordnet. Die flüssige Probe tritt durch die Eintrittsöffnung in den Kanal ein und füllt diesen zumindest bis das Testfeld benetzt wird, woraufhin bevorzugt ein oder mehrere Bestandteile der flüssigen Probe auf photometrischem oder elektrochemischem Wege analysiert werden. Ferner ist eine homogene Benetzung des Testfeldes mit einer durch den Kanaldurchmesser vorgegebenen Menge an Probe sichergestellt, wodurch die Präzision und Reproduzierbarkeit der Messung erhöht wird. Ein weiterer Vorteil des erfindungsgemäßen Testelementes ist, dass das Testfeld beim Öffnen (durch Stoßen, Aufschneiden, Abziehen) nicht beschädigt und durch abfallende Versiegelungsreste verunreinigt wird.

Die Versiegelung sollte bei dem erfindungsgemäßen Testelement aus einem weitgehend wasserdampfundurchlässigen Material bestehen, beispielsweise aus einer mit einem Polymer beschichteten Alufolie.

Erfindungsgemäß münden die Eintrittsöffnung und ggf. auch die Entlüftungsöffnung des Kanals in einen Freiraum in dem Testelement, der so angeordnet ist, dass er beim Öffnen der Versiegelung zur Umgebung des Testelements hin geöffnet wird. Vorzugsweise wird der Freiraum im unbenutzten Zustand des Testelements an einer ersten Seite zumindest teilweise durch die den Probenaufnahmeort verschließende Versiegelung und an einer der ersten Seite gegenüberliegenden zweiten Seite zumindest teilweise durch eine zweite Versiegelung begrenzt. Diese Anordnung hat den Vorteil, dass eine Durchstoßvorrichtung (zum Beispiel Lanzette, Nadel, Messer, Kanüle, Dorn) zunächst die zweite Versiegelung auf der dem Probenaufnahmeort gegenüberliegende Seite öffnen kann und anschließend durch den Freiraum hindurch auch die Versiegelung des Probenaufnahmeortes. Dies ermöglicht eine vereinfachte Konstruktion eines integrierten Messsystems, da der Aufnahmeort der flüssigen Probe und die Durchstoßvorrichtung auf gegenüberliegenden Seiten des Testelements angeordnet werden können und die Durchstoßvorrichtung der Probenaufnahme so nicht im Wege steht. Ferner kann die Durchstoßvorrichtung auch als Perforationsvorrichtung dienen, die außer dem Öffnen der beiden Versiegelungen auch das Perforieren der Haut eines Patienten zur Probenentnahme ausführt, so dass daraufhin aus dem Perforationsort der Haut austretende Körperflüssigkeit direkt als flüssige Probe in das Testelement eintreten kann. Der Patient muss dazu keine weiteren Handhabungsschritte oder Bewegungen durchführen und benötigt auch keine zusätzlichen Geräte.

In einer bevorzugten Ausführungsform der vorliegenden Erfindung verläuft der Kanal in dem Testelement u-förmig oder v-förmig. Dieser Verlauf hat den Vorteil einer Platz sparenden Unterbringung des Kanals in dem Testelement. Die Kanalenden können dabei in einem in dem Testelement enthaltenen Freiraum übereinander oder nebeneinander angeordnet sein.

Da für den bevorzugten Fall, dass der Kanal einen im Wesentlichen rechteckigen Querschnitt aufweist, eine Dimension, beispielsweise die Höhe des Kanals, durch die physikalischen Grenzen der Kapillaraktivität vorgegeben ist, lässt sich das Volumen des kapillaren Kanals durch geeignete Wahl der beiden übrigen Dimensionen, beispielsweise Länge und Breite, einstellen. Die Höhe liegt bevorzugt für wässrige flüssige Proben bei weniger als 1 mm, besonders bevorzugt bei weniger als 0,5 mm. Die Breite des Kanals kann bevorzugt weniger als 5 mm, besonders bevorzugt weniger als 2 mm und die Gesamtlänge des Kanals bevorzugt weniger als 5 cm, besonders bevorzugt weniger als 2 cm betragen.

In einer bevorzugten Ausführungsform der vorliegenden Erfindung ist der Abstand des Probenaufnahmeortes zur Eintrittsöffnung des Kanals geringer als der Abstand des Probenaufnahmeortes zur Entlüftungsöffnung des Kanals. Dies hat den Vorteil, dass die am Probenaufnahmeort in das Testelement eintretende flüssige Probe in die Eintrittsöffnung und nicht in die Entlüftungsöffnung des Kanals eintritt, da die Entlüftungsöffnung frei von Probenflüssigkeit bleiben soll, um ein Entweichen von Luft beim Befüllen des Kanals zu ermöglichen.

Vorzugsweise weist der Kanal im Bereich der Eintrittsöffnung eine bessere Benetzbarkeit durch die flüssige Probe auf als im Bereich der Entlüftungsöffnung. Durch diese Maßnahme soll ebenfalls ein Eintreten der flüssigen Probe in die Eintrittsöffnung des Kanals gefördert und in die Entlüflungsöffnung verhindert werden. Vorzugsweise weist außerdem der Kanal in seinem Inneren zwischen der Eintrittsöffnung und dem mindestens einen Testfeld eine bessere Benetzbarkeit durch die flüssige Probe auf, als zwischen dem mindestens einen Testfeld und der Entlüftungsöffnung. Dies kann durch eine Hydrophobierung der Entlüftungsöffnung und/oder sich daran anschließender, nicht zu benetzender Abschnitte des Kanals bewerkstelligt werden.

In einer bevorzugten Ausführungsform der vorliegenden Erfindung weist die den Probenaufnahmeort verschließende Versiegelung auf der der Innenseite des Testelements zugewandten Seite eine bessere Benetzbarkeit durch die flüssige Probe auf, als auf der der Innenseite des Testelements abgewandten Seite. Dies hat den Vorteil, dass beim direkten Aufbringen der flüssigen Probe auf die durchstoßene Versiegelung am Probenaufnahmeort die Außenseite der Versiegelung durch die Probe schlecht benetzt wird. Somit wird ein Transport der Probe zur besser zu benetzenden Innenseite der Versiegelung hin, und folglich der gewünschte Transport in das Testelement hinein gefordert.

In einer bevorzugten Ausführungsform der vorliegenden Erfindung ist in dem Testelement ein Trockenmittel enthalten. Dieses Trockenmittel kann den Schutz des Testelements vor eintretender Feuchtigkeit noch erhöhen, da es Feuchtigkeit adsorbiert. Das Trockenmittel kann beispielsweise in dem Kanal in der Nähe der Entlüftungsöffnung enthalten sein. Als Trockenmittel können zum Beispiel feste Trockenmittel verwendet werden. Vorzugsweise enthält das Trockenmittel Zeolithe oder Silikagel. Es sind Zeolithe oder Silikagel in Form von beads oder Tabletten, aber auch filmartig aufbringbare Schmelzkleber mit Silikagel oder Zeolithfüllung, wie sie in der Verpackungsindustrie zum Einsatz kommen, einsetzbar.

In einer bevorzugten Ausführungsform der vorliegenden Erfindung enthält das Testelement einen Waste-Bereich zur Aufnahme eines Überschusses an flüssiger Probe in dem Testelement. Der Waste-Bereich dient dazu, ein unerwünschtes Befüllen bestimmter Bereiche des Testelements, insbesondere des Kanals im Bereich der Entlüftungsöffnung, mit flüssiger Probe zu vermeiden.

Vorzugsweise weist die den Probenaufnahmeort verschließende Versiegelung eine Vorstrukturierung auf, die so gestaltet ist, dass beim Öffnen der Versiegelung ein definierter Grat entsteht. Der definierte Grat oder Auswurf soll dabei klare Kanten aufweisen, die einen Transport der flüssigen Probe in das Testelement hinein erleichtern. Durch die definierte Geometrie und Dimension des Grates oder Auswurfs wird erreicht, dass die flüssige Probe mit erhöhter Wahrscheinlichkeit mit dem kapillaraktiven Kanal in Kontakt kommt und in das Innere des Kanals eingesaugt wird. Eine mögliche Vorstrukturierung ist dabei kreuz- oder sternförmig, wobei ein umhüllendes Rechteck oder ein umhüllender Kreis zur Definition der Länge des Auswurfs hinzugefügt werden kann.

In einer bevorzugten Ausführungsform der vorliegenden Erfindung enthält das Testelement ein dem Testfeld zugewandtes Detektionsfenster zur photometrischen Analyse der flüssigen Probe auf dem Testfeld. Durch das Detektionsfenster, das im relevanten Wellenlängenbereich transparent ist, kann sowohl von einer Lichtquelle ausgesandtes Licht, als auch vom Testfeld reflektiertes Licht hindurchtreten. Ein Detektor kann so das reflektierte Licht detektieren, wobei die detektierten Signale durch eine Elektronik verarbeitet und das Ergebnis einem Benutzer auf einer Anzeigevorrichtung dargestellt werden können.

In einer bevorzugten Ausführungsform der vorliegenden Erfindung sind in mindestens einer den Kanal begrenzenden Wand Leiterbahnen für eine elektrochemische Analyse der flüssigen Probe auf dem Testfeld eingebracht. Diese Leiterbahnen werden für eine elektrochemische Analyse der flüssigen Probe im Testfeld des Testelements benötigt. Es ist auch eine gleichzeitige photometrische und elektrochemische Analyse denkbar.

In einer bevorzugten Ausführungsform der vorliegenden Erfindung ist das Testelement aus einer Vielzahl von Schichten aufgebaut Dies hat den Vorteil einer vereinfachten Herstellung. Die verschiedenen Eigenschaften, wie zum Beispiel die Benetzbarkeit, optische Transparenz, Stabilität und Form, verschiedene Bereiche des Testelements können durch die entsprechende Gestaltung der einzelnen Schichten erreicht werden, die miteinander verbunden werden.

Der Kanal kann bei dem schichtweise aufgebauten Testelement durch eine oder mehrere Schichten verlaufen. Der gesamte Kanal oder Teilbereiche des Kanals können durch Ausschneiden oder Ausstanzen eines Teils einer oder mehrerer Schichten hergestellt werden. Ein u- oder v-förmiger Kanal wird folglich durch Ausstanzen oder Ausschneiden eines u- oder v-förmigen Bereichs aus einer einzigen Zwischenschicht oder entsprechend geformter Bereiche aus mehreren, anschließend aufeinander gestapelten Schichten hergestellt (zum Beispiel zwei rechteckige Bereiche in zwei Zwischenschichten, zwischen denen eine Trägerschicht angeordnet wird, die die beiden rechteckigen Kanalabschnitte über eine Öffnung verbindet). Die Kanalhöhe wird dabei durch die Dicke der Zwischenschicht festgelegt. Ein Freiraum in dem Testelement kann ebenfalls durch Ausschneiden oder Ausstanzen von Bereichen der entsprechenden Form in einer oder mehreren Schichten erzeugt werden.

Das schichtweise aufgebaute erfindungsgemäße analytische Testelement umfasst mindestens eine Versiegelungsschicht. Eine erste Versiegelungsschicht dient als Versiegelung des Probenaufnahmeortes (mit oben beschriebenen Eigenschaften), die zur Verwendung des Testelements mittels Durchstoßen oder Aufschneiden (vorzugsweise mittels Durchstoßen) geöffnet wird. Weitere Versiegelungsschichten können beispielsweise auf der der ersten Versiegelungsschicht entgegengesetzten Seite des Testelements oder auf seinen in einem Winkel von 90° dazu angeordneten Seitenflächen zum Schutz des Testelements vor Feuchtigkeit und Verunreinigung dienen und gegebenenfalls teilweise zusätzlich zu der ersten Versiegelungsschicht bei der Verwendung des Testelements geöffnet werden.

In einer bevorzugten Ausführungsform der vorliegenden Erfindung weist die Versiegelungsschicht eine hydrophobe Beschichtung auf. Die erste Versiegelungsschicht hat vorzugsweise eine hydrophobe Beschichtung auf ihrer Außenseite, um die oben beschriebene bessere Benetzbarkeit durch die wässrige flüssige Probe auf ihrer Innenseite gegenüber ihrer Außenseite zu erreichen.

Vorzugsweise umfasst das schichtweise aufgebaute erfindungsgemäße Testelement mindestens eine Trägerschicht und mindestens eine, den Kanal zumindest teilweise enthaltende Zwischenschicht. Die Trägerschichten geben dem Testelement Stabilität. Sie können zum Beispiel den Kanal als Boden- und Deckschichten begrenzen und das Testfeld umfassen. Diese Boden- und Deckschichten können in ausgewählten Bereichen (vorzugsweise zwischen Eintrittsöffnung und Testfeld) hydrophile Materialien enthalten und/oder hydrophilierte Oberflächen aufweisen. Hydrophile Oberflächen sind in diesem Zusammenhang Wasser anziehende Flächen. Wässrige Proben, darunter auch Blut, spreiten auf solchen Oberflächen gut. Sie weisen eine gute Benetzbarkeit durch diese Proben auf. Solche Flächen sind u.a. dadurch charakterisiert, dass an der Grenzfläche ein Wassertropfen auf ihnen einen spitzen Rand- oder Kontaktwinkel ausbildet. Im Gegensatz dazu wird hydrophoben, d.h. Wasser abweisenden Oberflächen, an der Grenzfläche zwischen Wassertropfen und Oberfläche ein stumpfer Randwinkel ausgebildet.

Der Randwinkel als Resultat der Oberflächenspannungen der Prüfflüssigkeit und der zu untersuchenden Oberfläche ist als Maß für die Hydrophilie einer Oberfläche geeignet. Wasser hat beispielsweise eine Oberflächenspannung von 72 mN/m. Liegt der Wert der Oberflächenspannung der betrachteten Fläche weit, d.h. mehr als 20 mN/m unter diesem Wert, so ist die Benetzung schlecht und der resultierende Randwinkel ist stumpf. Eine solche Fläche wird als hydrophob bezeichnet. Nähert sich die Oberflächenspannung dem Wert, der für Wasser gefunden wird, so ist die Benetzung gut und der Randwinkel wird spitz. Wird die Oberflächenspannung dagegen gleich oder größer dem für Wasser gefundenen Wert, so zerläuft der Tropfen und es findet Totalspreitung der Flüssigkeit statt. Ein Randwinkel ist dann nicht mehr zu messen. Flächen, die mit Wassertropfen einen spitzen Randwinkel bilden oder bei denen Totalspreitung eines Wassertropfens beobachtet wird, werden als hydrophil bezeichnet.

Die Bereitschaft einer Kapillare, eine Flüssigkeit aufzusaugen, geht mit der Benetzbarkeit der Kanaloberfläche mit der Flüssigkeit einher. Für wässrige Proben bedeutet dies, dass eine Kapillare aus einem Material gefertigt werden sollte, dessen Oberflächenspannung nahe an 72 mN/m heranreicht oder diesen Wert übertrifft.

Ausreichend hydrophile Materialien zum Aufbau einer Kapillare, die schnell wässrige Proben aufsaugt, sind beispielsweise Glas, Metall oder Keramik. Für den Einsatz in Testelementen sind diese Materialien jedoch nicht so gut geeignet, da sie einige Nachteile aufweisen, beispielsweise Bruchgefahr bei Glas oder Keramik oder Veränderung der Oberflächeneigenschaften mit der Zeit bei zahlreichen Metallen. Üblicherweise werden deshalb zur Fertigung von Testelementen Kunststofffolien oder -formteil eingesetzt. Die verwendeten Kunststoffe übertreffen dabei in der Regel kaum eine Oberflächenspannung von 45 mN/m. Selbst mit den, relativ betrachtet, hydrophilsten Kunststoffen, wie beispielsweise Polymethylmethacrylat (PMMA) oder Polyamid (PA) lassen sich - wenn überhaupt - nur sehr langsam saugende Kapillaren aufbauen. Kapillaren aus unbehandelten hydrophoben Kunststoffen, wie beispielsweise Polystyrol (PS), Polypropylen (PP) oder Polyethylen (PE) saugen im Wesentlichen keine wässrigen Proben. Hieraus ergibt sich die Notwendigkeit, Kunststoffe für die Verwendung als Konstruktionsmaterial für Testelemente mit kapillaraktiven Kanälen hydrophil auszustatten, d.h. zu hydrophilieren.

In einer bevorzugten Ausführungsform des erfindungsgemäßen analytischen Testelements ist zumindest eine, besser jedoch zwei, ganz besonders bevorzugt zwei sich gegenüberliegende Flächen der die innere Oberfläche des zum kapillaren Flüssigkeitstransports befähigten Kanals bildenden Flächen, hydrophiliert. Wird mehr als eine Fläche hydrophiliert, so können die Flächen entweder mit der gleichen oder mit unterschiedlichen Methoden hydrophil gemacht werden. Die Hydrophilierung ist vor allem dann notwendig, wenn die Materialien, die den kapillaraktiven Kanal bilden, insbesondere die Trägerschichten, selbst hydrophob oder nur wenig hydrophil sind, beispielsweise weil sie aus unpolaren Kunststoffen bestehen.

Idealerweise wird die Hydrophilierung der Oberfläche des kapillaren Kanals dadurch erreicht, dass zu seiner Fertigung, insbesondere zur Fertigung der Trägerschichten, ein hydrophiles Material eingesetzt wird, das jedoch die Probenflüssigkeit selbst nicht oder nicht wesentlich aufzusaugen vermag. Wo dies nicht möglich ist, kann die Hydrophilierung einer hydrophoben oder nur sehr wenig hydrophilen Oberfläche durch geeignete Beschichtung mit einer stabilen, gegenüber dem Probenmaterial inerten, hydrophilen Schicht erreicht werden, beispielsweise durch kovalente Bindung von photoreaktiv ausgerüsteten, hydrophilen Polymeren auf eine Kunststoffoberfläche, durch Aufbringen netzmittelhaltiger Schichten oder durch Beschichtung von Oberflächen mit Nanokompositen mittels Sol-Gel-Technologie. Darüber hinaus ist es möglich, durch thermische, physikalische oder chemische Behandlung der Oberfläche eine gesteigerte Hydrophilie zu erzielen.

Ganz besonders bevorzugt wird die Hydrophilierung durch die Verwendung von dünnen Schichten oxidierten Aluminiums erreicht. Diese Schichten werden entweder direkt auf die gewünschten Bauteile des Testelements aufgebracht, beispielsweise durch Vakuumbedampfen der Werkstücke mit metallischem Aluminium und anschließender Oxidation des Metalls, oder in Form von Metallfolien oder metallbeschichteten Kunststofffolien für den Testelement-Aufbau verwendet, die ebenfalls zur Erzielung der erwünschten Hydrophilie oxidiert werden müssen. Metallschichtdicken von 1 bis 500 nm sind dabei ausreichend. Die Metallschicht wird anschließend zur Bildung der oxidierten Form oxidiert, wobei sich neben der elektrochemischen, anodischen Oxidation vor allem die Oxidation in Gegenwart von Wasserdampf oder durch Kochen in Wasser als besonders geeignete Methoden herausgestellt haben. Die so erzielten Oxidschichten sind je nach Methode zwischen 0,1 und 500 nm, bevorzugt zwischen 10 und 100 nm dick. Größere Schichtdicken sowohl der Metallschicht als auch der Oxidschicht sind ebenfalls praktisch realisierbar.

Die als Boden- und Deckschichten des Kanals dienenden Trägerschichten können außerdem in ausgewählten Bereichen (vorzugsweise zwischen Entlüftungsöffnung und Testfeld) hydrophobe Materialien aufweisen oder eine hydrophobe Oberflächenbeschichtung besitzen. Diese soll für eine schlechte Benetzbarkeit der Trägerschichtoberfläche durch wässrige flüssige Proben sorgen, um zum Beispiel ein Eindringen der Proben in den Bereich des Kanals bei der Entlüftungsöffnung und ein Zusetzen derselben mit Proben zu vermeiden.

Als Zwischenschicht wird im Zusammenhang mit dieser Erfindung eine zwischen zwei Trägerschichten angeordnete Schicht bezeichnet, die den Kanal zumindest teilweise enthält und deren Dicke die kapillaraktive Höhe zumindest von Teilen des Kanals bestimmt. Zwischenschichten können doppelseitige adhäsive Bänder sein oder direkt oder unter Einsatz von Haftvermittlern mit den als Boden- oder Deckschichten dienenden Trägerschichten verbunden werden. Die Zwischenschichten können mit hydrophoben Füllstoffen versehen werden, welche ihre Wasserdampfdurchlässigkeit herabsetzen, damit der Kanal vor über die Zwischenschichten eintretender Feuchtigkeit geschützt wird. Alternativ dazu können die Zwischenschichten selbst aus hydrophoben Materialien gefertigt sein.

In einer besonders bevorzugten Ausführungsform der vorliegenden Erfindung sind die Schichten in folgender Reihenfolge angeordnet:
A) eine den Probenaufnahmeort umfassende erste Versiegelungsschicht,
B) eine transparente erste Trägerschicht,
C) eine einen ersten Teil des Kanals enthaltende erste Zwischenschicht,
D) eine eine Öffnung enthaltende zweite Trägerschicht,
E) eine einen zweiten Teil des Kanals enthaltende zweite Zwischenschicht,
F) eine dritte Trägerschicht und
G) eine zweite Versiegelungsschicht,
wobei die Öffnung in der zweiten Trägerschicht den ersten Teil des Kanals mit dem zweiten Teil des Kanals verbindet und ein Testfeld in dem ersten Teil des Kanals angeordnet ist und sich bei dem unbenutzten Testelement ein Freiraum durch die Schichten B) bis F) erstreckt, in den der erste und der zweite Teil des Kanals münden. Dabei können die erste Versiegelungsschicht auf ihrer Außenseite und die dem zweiten Teil des Kanals zugewandten Seiten der zweiten und der dritten Trägerschicht eine hydrophobe Beschichtung aufweisen. In dem zweiten Teil des Kanals kann ein Trockenmittel angeordnet sein. Die erste Versiegelungsschicht kann ein Detektionsfenster zur photometrischen Analyse der flüssigen Probe auf dem Testfeld enthalten. Die Zwischenschichten sind vorzugsweise hydrophob. Vorzugsweise sind die dem ersten Teil des Kanals zugewandten Seiten der ersten und zweiten Trägerschicht hydrophil oder hydrophil beschichtet.

In einer bevorzugten Ausführungsform der vorliegenden Erfindung ist die Versiegelung oder die Versiegelungsschicht eine Verbundfolie, die eine Alufolie, eine Außenschicht aus PE, PET oder orientiertem PA und eine Innenschicht aus PE, PP oder Lacken umfasst oder die einen Hochbarriereverbund aus PET/SiOx umfasst.

Die Erfindung bezieht sich ferner auf ein Testelement-Magazin, das als Band, Trommel, Stapel oder Diskette ausgebildet ist In einem Band hängen viele erfindungsgemäße Testelemente aneinander, so dass sie zum Beispiel auf einer Spule aufgewickelt werden können. Durch ein Drehen der Spule kann jeweils ein neues Testelement in eine Arbeitsposition (Probenentnahme und/oder Messposition) gebracht werden. Dabei ist ein manueller oder automatischer Antrieb der Spule möglich.

Ein als Trommel ausgebildetes Testelement-Magazin hat bevorzugt die Form einer im Wesentlichen zylindrischen länglichen Trommel, in der Kammern zur Aufnahme der Testelemente sternförmig um die Längsachse der Trommel angeordnet sind. Die Länge der Trommel richtet sich im Wesentlichen nach der Länge der darin unterzubringenden Testelemente. Die Grund- und Deckelflächen der zylindrischen Trommel enthalten die Öffnungen der Kammern, welche aufgrund der erfindungsgemäß vorhandenen einzelnen Versiegelung der Testelemente nicht verschlossen sein müssen. Das Testelement sollte jedoch durch entsprechende Vorkehrungen bis zu seiner Entnahme aus der Trommel in der Kammer rutschfest gehalten werden.

In einem Stapel liegen die einzelnen Testelemente in einem Vorratsbehältnis übereinander und können einzeln in die entsprechende Messposition gebracht werden. Das den Stapel enthaltende Vorratsbehältnis braucht bei der vorliegenden Erfindung keine weiteren Vorkehrungen zum Schutz der Testelemente zu treffen, da diese bereits erfindungsgemäß jeweils eine Versiegelung aufweisen.

Bei einer Diskette sind die einzelnen Testelemente radial in einem definierten Abstand zueinander auf einer im Wesentlichen runden Scheibe angeordnet Durch Drehen der Diskette kommt jeweils ein neues Testelement in eine Arbeitsposition, wobei das Drehen manuell oder automatisch erfolgen kann.

Die Erfindung bezieht sich weiterhin auf ein System zur Analyse flüssiger Proben mit mindestens einem erfindungsgemäßen Testelement-Magazin und einer Durchstoßvorrichtung zum Öffnen der Versiegelung jeweils eines Testelements am Probenaufnahmeort kurz vor der Aufnahme einer flüssigen Probe und mit einem Detektor zur Analyse der flüssige Probe im Testfeld eines Testelements. Der Detektor ist dabei zum Beispiel ein Gerät zur photometrischen oder elektrochemischen Auswertung von Testelementen. Unter einer Durchstoßvorrichtung wird in diesem Zusammenhang eine Vorrichtung verstanden, die die Versiegelung jeweils eines erfindungsgemäßen Testelements durch einen Stoß oder Schnitt öffnen kann. Sie weist spitze oder scharfe Kanten für diesen Zweck auf. Es handelt sich beispielsweise um einen Dorn, eine Nadel, eine Lanzette, ein Messer oder eine Kanüle.

In einer Ausführungsform der vorliegenden Erfindung enthält das System zusätzlich zu der Durchstoßvorrichtung eine Perforationsvorrichtung in Form einer Lanzette, einer Kanüle, einer Nadel, eines Messers oder eines Dorns zum Erzeugen einer Perforation in der Haut eines Patienten, um eine Körperflüssigkeit als flüssige Probe zu entnehmen. Es kann aber auch die Durchstoßvorrichtung selbst als Perforationsvorrichtung dienen. Die Perforationsvorrichtung dient dazu, eine Körperöffnung zu erzeugen, durch die eine Körperflüssigkeit austreten kann. In dem Fall, dass die Durchstoßvorrichtung selbst als Perforationsvorrichtung dient, ist es vorteilhaft, wenn das Testelement mindestens zwei Versiegelungen auf gegenüberliegenden Seiten des Testelements enthält, die eine geringe Durchstoßkraft erfordern, und ein Freiraum zwischen diesen Versiegelungen in dem Testelement existiert, so dass die als Perforationsvorrichtung dienende Durchstoßvorrichtung durch das Testelement (und den Freiraum) hindurchstoßbar und in die Hautoberfläche eines Benutzers einstechbar ist Dann kann die aus der Hautoberfläche austretende Körperflüssigkeit direkt den Probenaufnahmeort des Testelements beaufschlagen.

In dem Fall zweier getrennter Durchstoß- und Perforationsvorrichtungen kann die Perforationsvorrichtung von der Durchstoßvorrichtung konzentrisch umgeben sein. Dies ermöglicht eine Platz sparende Unterbringung beider Vorrichtungen in dem erfindungsgemäßen Analysesystem. Ein weiterer Vorteil dieser Anordnung ist, dass sich das Testelement für beide Vorrichtungen gleichzeitig in Arbeitsposition befindet.

In einer weiteren Ausführungsform der vorliegenden Erfindung sind die Durchstoßvorrichtung und die Perforationsvorrichtung in einer Halterung nebeneinander angeordnet und durch eine Drehung der Halterung nacheinander in eine Arbeitsposition bringbar.

Vorzugsweise ist das Testelement-Magazin ein Band aus aneinander gereihten, einzeln versiegelten Testelementen, wobei das System eine Transporteinrichtung umfasst, die so ausgelegt ist, dass sie nacheinander jeweils ein Testelement in eine Arbeitsposition transportieren kann, in der die Versiegelung des Testelements durch die Durchstoßvorrichtung durchstoßen werden kann. In einer anderen bevorzugten Ausführungsform ist das Testelement-Magazin eine Diskette, auf der die Testelemente radial angeordnet sind, wobei jeweils ein Testelement durch Drehung der Diskette in eine Arbeitsposition drehbar ist, in der die Versiegelung des Testelements durch die Durchstoßvorrichtung geöffnet werden kann.

Vorzugsweise umfasst das erfindungsgemäße System eine Kompressionseinheit zur Erhöhung des Drucks auf die perforierte Haut des Patienten bei der Entnahme der Körperflüssigkeit. Diese Kompressionseinheit dient zum Hinausbefördern der Körperflüssigkeit aus der Körperöffnung. Es kann sich dabei um eine Kompressionseinheit handeln, wie sie in WO 01/89383 offenbart ist. Der Anwender drückt das Körperteil an der Entnahmestelle an die gegebenenfalls deformierbare Kompressionseinheit. In diesem angedrückten Zustand belässt er das Körperteil während des Erzeugens einer Hautöffnung und/oder während der Gewinnung der Körperflüssigkeit.

Die Erfindung bezieht sich weiterhin auf die Verwendung eines erfindungsgemäßen analytischen Testelements zur Analyse des Glukosegehalts im Blut oder in interstitieller Flüssigkeit.

Die Erfindung wird anhand der Zeichnung nachstehend näher erläutert.

Es zeigt:
- Figur 1: den Querschnitt eines erfindungsgemäßen analytischen Testelements in ver- siegeltem, geöffnetem und mit flüssiger Probe beaufschlagtem Zustand,
- Figur 2: den Querschnitt eines erfindungsgemäßen analytischen Testelements mit einem Waste-Bereich,
- Figur 3: ein erfindungsgemäßes System zur Analyse flüssiger Proben mit einem er- findungsgemäßen Testelement-Magazin in Form eines Bandes, einer Durch- stoßvorrichtung und einem Detektor und
- Figur 4: ein erfindungsgemäßes System zur Analyse flüssiger Proben mit einem er- findungsgemäßen Testelement-Magazin in Form einer Diskette.

### Besondere Ausführungsformen

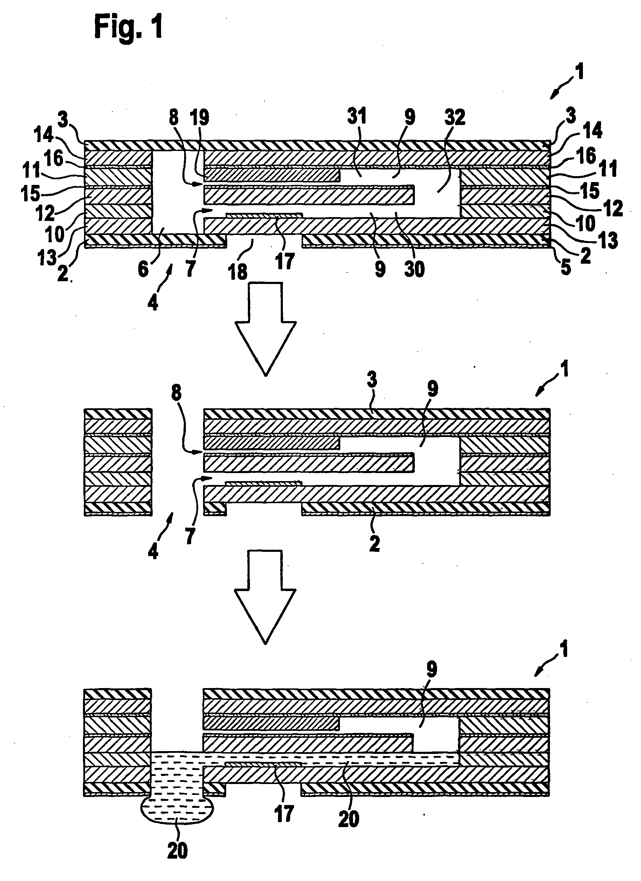

Figur 1 zeigt ein erfindungsgemäßes analytisches Testelement in versiegeltem, geöffnetem und mit flüssiger Probe beaufschlagtem Zustand.

Die obere Abbildung in Figur 1 zeigt das Testelement 1 in versiegeltem Zustand. Es ist aus einer Vielzahl von Schichten aufgebaut. An beiden Außenseiten wird es durch eine erste und eine zweite Versiegelungsschicht (2 beziehungsweise 3) versiegelt, die eine geringe Wasserdampfdurchlässigkeit aufweisen. Die erste Versiegelungsschicht 2 verschließt den Probenaufnahmeort 4 und hat an ihrer Außenseite eine hydrophobe Beschichtung 5, damit eine wässrige flüssige Probe nicht die Außenseite benetzt, sondern bei geöffneter Versiegelung am Probenaufnahmeort 4 in das Testelement 1 hineingelangt. In dem Testelement 1 ist ein Freiraum 6 enthalten, in den eine Eintrittsöffnung 7 und eine Entlüftungsöffnung 8 eines zum kapillaren Transport einer flüssigen Probe geeigneten Kanals 9 münden. Der Kanal 9 ist u-förmig und erstreckt sich über mehrere Schichten, nämlich zwei Zwischenschichten 10, 11 (erster und zweiter Teil des Kanals 30, 31) und eine Trägerschicht 12 (Öffnung 32). Außer dieser zweiten Trägerschicht 12 enthält das Testelement 1 noch eine transparente erste Trägerschicht 13 und eine angrenzend an die zweite Versiegelungsschicht 3 angeordnete dritte Trägerschicht 14. Die Trägerschichten 12, 13, 14 bilden die Deck- und Bodenschichten des Kanals 9. Die zweite und dritte Trägerschicht 12, 14 weisen jeweils auf ihrer, dem Kanal 9 zugewandten Seite eine hydrophobe Beschichtung 15 beziehungsweise 16 auf, die die wässrige flüssige Probe davon abhalten soll, in den der Entlüftungsöffnung 8 zugewandten Teil des Kanals 9 einzutreten. In dem Kanal 9 ist auf der der transparenten ersten Trägerschicht 13 zugewandten Seite ein Testfeld 17 angeordnet, auf dem die flüssige Probe analysiert werden kann. Auf der dem Testfeld 17 gegenüberliegenden Seite der transparenten ersten Trägerschicht 13 befindet sich ein Detektionsfenster 18 in der ersten Versiegelungsschicht 2 für die photometrische Analyse der flüssigen Probe auf dem Testfeld 17. In dem Kanal 9 ist ferner nahe der Entlüftungsöffung 8 ein Trockenmittel platziert, das eine mögliche Restfeuchtigkeit im versiegelten Testelement 1 aufnehmen soll.

Die mittlere Abbildung der Figur 1 zeigt ein geöffnetes Testelement 1, bei dem beide Versiegelungsschichten 2, 3 im Bereich des Freiraums 6 durchstoßen oder aufgeschnitten sind. Der Probenaufnahmeort 4 ist nun geöffnet und bereit, eine flüssige Probe aufzunehmen. Durch das Öffnen der ersten Versiegelungsschicht 2 am Probenaufnahmeort 4 sind auch gleichzeitig die Eintrittsöffnung 7 des Kanals 9 und die Entlüftungsöffnung 8 des Kanals 9 zur Umgebung des Testelements 1 hin geöffnet worden.

Die untere Abbildung der Figur 1 zeigt, wie die flüssige Probe 20 zum Testfeld 17 gelangt. Am Probenaufnahmeort 4 wird ein ausreichend großer Tropfen der flüssigen Probe 20 auf das Testelement 1 gegeben, von wo aus die flüssige Probe 20 durch den Freiraum 6 zur Eintrittsöffnung 7 des Kanals 9 gelangt und durch Kapillarkräfte in diesen eingesaugt wird. In dem Kanal 9 fließt die Probe 20 über das Testfeld 17, wo sie analysiert wird.

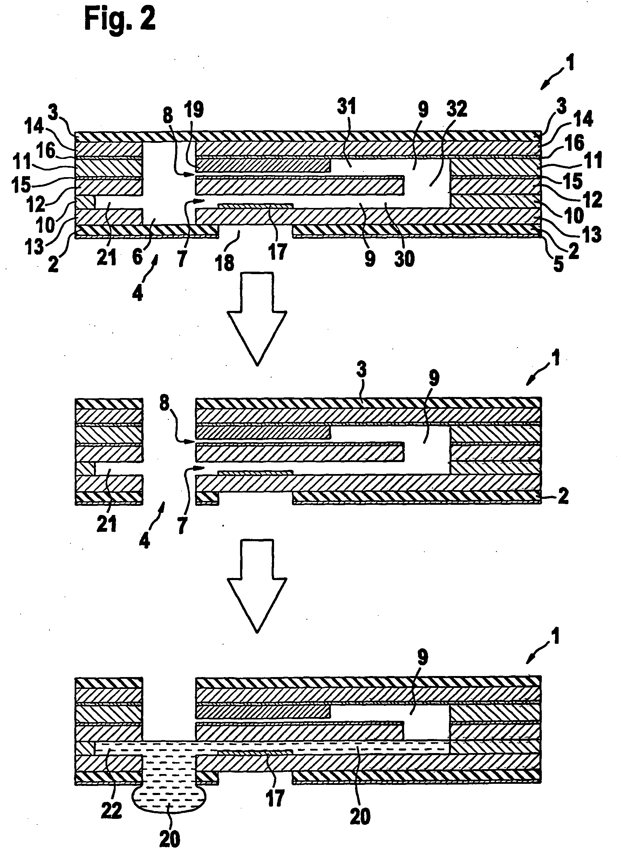

Figur 2 zeigt ein weiteres erfindungsgemäßes analytisches Testelement, dessen Aufbau dem in Figur 1 dargestellten Testelement entspricht und das zusätzlich einen Waste-Bereich 21 aufweist, der einen Überschuss 22 an flüssiger Probe 20 aufnehmen kann. Bezüglich des restlichen Aufbaus trifft die zu Figur 1 aufgeführte Beschreibung auch auf das Testelement 1 gemäß Figur 2 zu und die Bezugszeichen haben die gleiche Bedeutung wie in Figur 1.

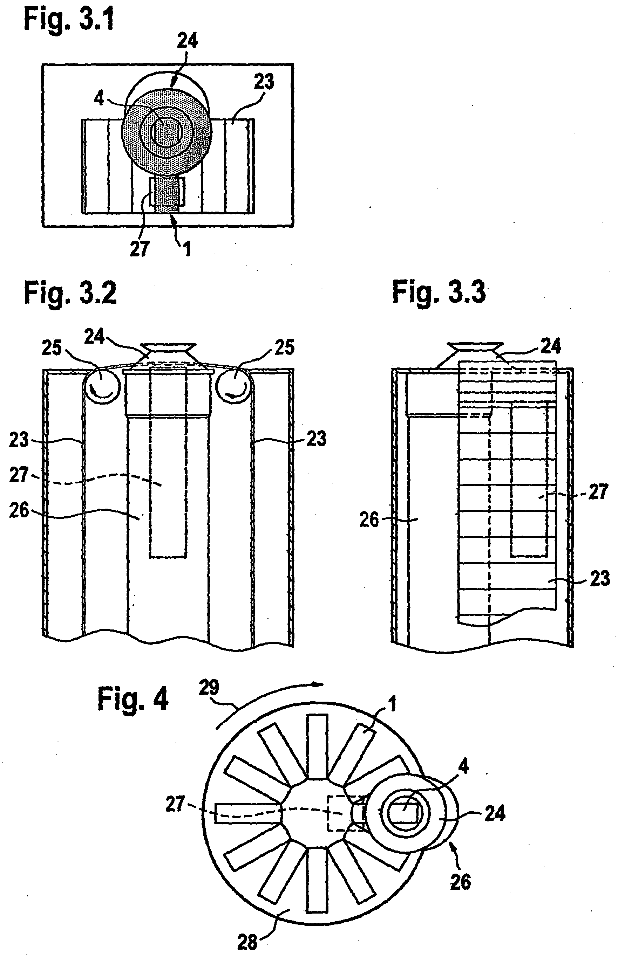

Figur 3 zeigt schematisch ein erfindungsgemäßes System zur Analyse flüssiger Proben.

Die linke obere Darstellung in Figur 3 (Fig. 3.1) zeigt schematisch eine Draufsicht auf das System. Die erfindungsgemäßen Testelemente 1 liegen als Magazin in Form eines Bandes 23 vor. Ein Expressionskonus dient als Kompressionseinheit 24 zum Erhöhen des Drucks auf die die Körperöffnung umgebende Haut eines Patienten zur Förderung einer Körperflüssigkeit wie Blut oder interstitieller Flüssigkeit aus der Körperöffnung. Die Kompressionseinheit 24 ist dabei über dem Probenaufnahmeort 4 eines sich in Arbeitsposition befindenden Testelements 1 angeordnet.

Die linke untere Darstellung in Figur 3 (Fig. 3.2) zeigt schematisch eine erste Seitenansicht desselben erfindungsgemäßen Systems. Das Magazin in Form eines Bandes 23 läuft über zwei Transportrollen 25, um jeweils ein Testelement 1 in Arbeitsposition zu bringen, in der sich die Kompressionseinheit 24 direkt über dem Probenaufnahmeort befindet. In der in Figur 3 dargestellten zylindrischen Anordnung 26 sind (nicht sichtbar) eine Durchstoßvorrichtung und gegebenenfalls eine Perforationsvorrichtung angeordnet, die zum Beispiel durch Drehen einer sie haltenden Halterung nacheinander in Arbeitsposition gebracht werden können, also in eine Position, in der die Durchstoßvorrichtung die Versiegelung auf beiden Seiten des Testelements 1 (insbesondere am Probenaufnahmeort) Durchstechen, und die Perforationsvorrichtung eine Körperöffnung in einem auf der Kompressionseinheit 24 platzierten Körperteil erzeugen kann. Vor der zylindrischen Anordnung befindet sich ein Detektor 27, zum Beispiel zur photometrischen Analyse einer Probe, der auf ein (nicht dargestelltes) Detektionsfenster in dem Testelement 1 in Arbeitsposition gerichtet ist.

Die rechte untere Darstellung in Figur 3(Fig. 3.3) zeigt schematisch eine zweite Seitenansicht desselben erfindungsgemäßen Systems. Es sind noch einmal gut die zylindrische Anordnung 26, der Detektor 27, das bandförmige Magazin 23 und die Kompressionseinheit 24 erkennbar.

Figur 4 zeigt schematisch ein weiteres erfindungsgemäßes System zur Analyse flüssiger Proben in einer Draufsicht.

Das System enthält ein Magazin in Form einer Diskette 28, auf dem die erfindungsgemäßen Testelemente 1 radial auf einer im Wesentlichen runden Scheibe angeordnet sind. Durch Drehung des Magazins 28 in Drehrichtung 29 kann jeweils ein Testelement 1 in Arbeitsposition gebracht werden (Probenaufnahmeort 4 unter der Kompressionseinheit 24). Das System enthält ferner eine (nicht dargestellte) Durchstoßvorrichtung, die in einer zylindrischen Anordnung 26 untergebracht ist und einen Detektor 27.

### Bezugszeichenliste

- 1: Testelement
- 2: erste Versiegelungsschicht
- 3: zweite Versiegelungsschicht
- 4: Probenaufnahmeort
- 5: hydrophobe Beschichtung
- 6: Freiraum
- 7: Eintrittsöffnung
- 8: Entlüftungsöffnung
- 9: Kanal
- 10: erste Zwischenschicht
- 11: zweite Zwischenschicht
- 12: zweite Trägerschicht
- 13: erste Trägerschicht
- 14: dritte Trägerschicht
- 15: hydrophobe Beschichtung
- 16: hydrophobe Beschichtung
- 17: Testfeld
- 18: Detektionsfenster
- 19: Trockenmittel
- 20: flüssige Probe
- 21: Waste-Bereich
- 22: Überschuss an flüssiger Probe
- 23: Magazin in Form eines Bandes
- 24: Kompressionseinheit
- 25: Transportrollen
- 26: zylindrische Anordnung
- 27: Detektor
- 28: Magazin in Form einer Diskette
- 29: Drehrichtung
- 30: erster Teil des Kanals
- 31: zweiter Teil des Kanals
- 32: Öffnung

## Patentansprüche

1. Analytisches Testelement zur Analyse einer flüssigen Probe (20) enthaltend einen zum kapillaren Transport der flüssigen Probe (20) geeigneten Kanal (9) mit einer Eintrittsöffnung (7) für die flüssige Probe (20) und einer Entlüftungsöffnung (8), wobei in dem Kanal (9) beabstandet zu der Eintrittsöffnung (7) mindestens ein Testfeld (17) angeordnet ist und wobei das Testelement (1) einen mit einer Versiegelung verschlossenen Probenaufnahmeort (4) umfasst, der so ausgelegt ist, dass durch ein Öffnen der Versiegelung gleichzeitig der Probenaufnahmeort (4) und die Eintrittsöffnung (7) des Kanals (9) zur Umgebung des Testelements (1) hin öffenbar ist und die flüssige Probe (20) durch das Testelement (1) über den Probenaufnahmeort (4) und die Eintrittsöffnung (7) in den Kanal (9) zur Analyse in dem Testfeld (17) aufnehmbar ist, wobei durch ein Öffnen der Versiegelung durch Durchstoßen oder Aufschneiden gleichzeitig der Probenaufnahmeort (4), die Eintrittsöffnung (7) des Kanals (9) und die Entlüftungsöffnung (8) des Kanals (9) zur Umgebung des Testelements (1) hin öffenbar sind, **dadurch gekennzeichnet, dass** die Eintrittsöffnung (7) und die Entlüftungsöffnung (8) des Kanals (9) in einen Freiraum (6) in dem Testelement (1) münden, der so angeordnet ist, dass er beim Öffnen der Versiegelung zur Umgebung des Testelements (1) hin geöffnet wird.

2. Analytisches Testelement gemäß Anspruch 1, **dadurch gekennzeichnet, dass** die Eintrittsöffnung (7) an einem Ende des Kanals (9) und die Entlüftungsöffnung (8) am anderen Ende des Kanals (9) angeordnet ist.

3. Analytisches Testelement gemäß Anspruch 1, **dadurch gekennzeichnet, dass** der Freiraum (6) im unbenutzten Zustand des Testelements (1) an einer ersten Seite zumindest teilweise durch die den Probenaufnahmeort (4) verschießende Versiegelung und an einer der ersten Seite gegenüberliegenden zweiten Seite zumindest teilweise durch eine zweite Versiegelung begrenzt wird.

4. Analytisches Testelement gemäß einem der Ansprüche 1 bis 3, **dadurch gekennzeichnet, dass** der Kanal (9) in dem Testelement (1) u-förmig oder v-förmig verläuft.

5. Analytisches Testelement gemäß einem der Ansprüche 1 bis 4, **dadurch gekennzeichnet, dass** der Kanal (9) einen im Wesentlichen rechteckigen Querschnitt und eine Breite von < 5 mm und eine Höhe von < 1 mm aufweist.

6. Analytisches Testelement gemäß einem der Ansprüche 1 bis 5, **dadurch gekennzeichnet, dass** der Kanal (9) eine Gesamtlänge von < 5 cm aufweist.

7. Analytisches Testelement gemäß einem der Ansprüche 1 bis 6, **dadurch gekennzeichnet, dass** der Abstand des Probenaufnahmeortes (4) zur Eintrittsöffnung (7) des Kanals (9) geringer ist als der Abstand des Probenaufnahmeortes (4) zur Entlüftungsöffnung (8) des Kanals (9).

8. Analytisches Testelement gemäß einem der Ansprüche 1 bis 7, **dadurch gekennzeichnet, dass** der Kanal (9) im Bereich der Eintrittsöffnung (7) eine bessere Benetzbarkeit durch die flüssige Probe aufweist, als im Bereich der Entlüftungsöffnung (8).

9. Analytisches Testelement gemäß einem der Ansprüche 1 bis 8, **dadurch gekennzeichnet, dass** der Kanal (9) in seinem Inneren zwischen der Eintrittsöffnung (7) und dem mindestens einen Testfeld (17) eine bessere Benetzbarkeit durch die flüssige Probe (20) aufweist, als zwischen dem mindestens einen Testfeld (17) und der Entlüftungsöffnung (8).

10. Analytisches Testelement gemäß einem der Ansprüche 1 bis 9, **dadurch gekennzeichnet, dass** die den Probenaufnahmeort (4) verschließende Versiegelung auf der der Innenseite des Testelements (1) zugewandten Seite eine bessere Benetzbarkeit durch die flüssige Probe (20) als auf der der Innenseite des Testelements (1) abgewandten Seite aufweist.

11. Analytisches Testelement gemäß einem der Ansprüche 1 bis 10, **dadurch gekennzeichnet, dass** in dem Testelement (1) ein Trockenmittel (19) enthalten ist.

12. Analytisches Testelement gemäß Anspruch 11, **dadurch gekennzeichnet, dass** das Trockenmittel (19) Zeolithe oder Silikagel enthält.

13. Analytisches Testelement gemäß einem der Ansprüche 1 bis 12, **gekennzeichnet durch** einen Waste-Bereich (21) zur Aufnahme eines Überschusses (22) an flüssiger Probe (20) in dem Testelement (1).

14. Analytisches Testelement gemäß einem der Ansprüche 1 bis 13, **dadurch gekennzeichnet, dass** die den Probenaufnahmeort (4) verschließende Versiegelung eine Vorstrukturierung aufweist, die so gestaltet ist, dass beim Öffnen der Versiegelung ein definierter Grat entsteht.

15. Analytisches Testelement gemäß Anspruch 14, **dadurch gekennzeichnet, dass** die Vorstrukturierung kreuzförmig oder sternförmig ist.

16. Analytisches Testelement gemäß einem der Ansprüche 1 bis 15, **dadurch gekennzeichnet, dass** das Testelement (1) ein dem Testfeld (17) zugewandtes Detektionsfenster (18) zur photometrischen Analyse der flüssigen Probe (20) auf dem Testfeld (17) enthält.

17. Analytisches Testelement gemäß einem der Ansprüche 1 bis 16, **dadurch gekennzeichnet, dass** in mindestens einer den Kanal (9) begrenzenden Wand Leiterbahnen für eine elektrochemische Analyse der flüssigen Probe (20) auf dem Testfeld (17) eingebracht sind.

18. Analytisches Testelement gemäß einem der Ansprüche 1 bis 17, **dadurch gekennzeichnet, dass** das Testelement (1) aus einer Vielzahl von Schichten aufgebaut ist.

19. Analytisches Testelement gemäß Anspruch 18, **dadurch gekennzeichnet, dass** der Kanal (9) durch eine oder mehrere Schichten verläuft.

20. Analytisches Testelement gemäß einem der Ansprüche 18 oder 19, **dadurch gekennzeichnet, dass** das Testelement (1) mindestens eine Versiegelungsschicht (2, 3) umfasst.

21. Analytisches Testelement gemäß Anspruch 20, **dadurch gekennzeichnet, dass** die Versiegelungsschicht (2, 3) eine hydrophobe Beschichtung (5) aufweist.

22. Analytisches Testelement gemäß einem der Ansprüche 18 bis 21, **dadurch gekennzeichnet, dass** das Testelement (1) mindestens eine Trägerschicht (12, 13, 14) und mindestens eine den Kanal (9) zumindest teilweise enthaltende Zwischenschicht (10, 11) umfasst.

23. Analytisches Testelement gemäß einem der Ansprüche 18 bis 22, **dadurch gekennzeichnet, dass** die Schichten in folgender Reihenfolge angeordnet sind:
A) eine den Probenaufnahmeort (4) umfassende erste Versiegelungsschicht (2),
B) eine transparente erste Trägerschicht (13),
C) eine einen ersten Teil (30) des Kanals (9) enthaltende erste Zwischenschicht (10),
D) eine eine Öffnung (32) enthaltende zweite Trägerschicht (12),
E) eine einen zweiten Teil (31) des Kanals (9) enthaltende zweite Zwischenschicht (11),
F) eine dritte Trägerschicht (14) und
G) eine zweite Versiegelungsschicht (3),
wobei die Öffnung (32) in der zweiten Trägerschicht (12) den ersten Teil (30) des Kanals (9) mit dem zweiten Teil (31) des Kanals (9) verbindet und ein Testfeld (17) in dem ersten Teil des Kanals (9) angeordnet ist, und sich bei dem unbenutzten Testelement (1) ein Freiraum (6) durch die Schichten B) bis F) erstreckt, in den der erste und der zweite Teil (30, 31) des Kanals (9) münden

24. Analytisches Testelement gemäß Anspruch 23, **dadurch gekennzeichnet, dass** die erste Versiegelungsschicht (2) auf ihrer Außenseite und die dem zweiten Teil (31) des Kanals (9) zugewandten Seiten der zweiten und der dritten Trägerschicht (12, 14) eine hydrophobe Beschichtung (15, 16) aufweisen.

25. Analytisches Testelement gemäß einem der Ansprüche 23 oder 24, **dadurch gekennzeichnet, dass** in dem zweiten Teil (31) des Kanals (9) ein Trockenmittel (19) angeordnet ist.

26. Analytisches Testelement gemäß einem der Ansprüche 23 bis 25, **dadurch gekennzeichnet, dass** die erste Versiegelungsschicht (2) ein Detektionsfenster (18) zur photometrischen Analyse der flüssigen Probe (20) auf dem Testfeld (17) enthält.

27. Analytisches Testelement gemäß einem der Ansprüche 23 bis 26, **dadurch gekennzeichnet, dass** die Zwischenschichten (10, 11) hydrophob sind.

28. Analytisches Testelement gemäß einem der Ansprüche 23 bis 27, **dadurch gekennzeichnet, dass** die dem ersten Teil (30) des Kanals (9) zugewandten Seiten der ersten und zweiten Trägerschicht (13, 12) hydrophil oder hydrophil beschichtet sind.

29. Analytisches Testelement gemäß einem der Ansprüche 1 bis 28, **dadurch gekennzeichnet, dass** die Versiegelung oder die Versiegelungsschicht (2, 3) eine Verbundfolie ist, die eine Alufolie, eine Außenschicht aus PE, PET oder orientiertem PA und eine Innenschicht aus PE, PP oder Lacken umfasst oder die einen Hochbarriereverbund aus PET/SiOₓ umfasst.

30. Testelement-Magazin mit einer Vielzahl von Testelementen (1) nach einem der Ansprüche 1 bis 29, wobei das Testelement-Magazin als Band (23), Trommel, Stapel oder Diskette (28) ausgebildet ist.

31. System zur Analyse flüssiger Proben (20) mit mindestens einem analytischen Testelement (1) nach einem der Ansprüche 1 bis 29 und einer Durchstoßvorrichtung zum Öffnen der Versiegelung jeweils eines Testelements (1) am Probenaufnahmeort (4) kurz vor der Aufnahme einer flüssigen Probe (20) und mit einem Detektor (25) zur Analyse der flüssigen Proben (20) im Testfeld (17) eines Testelements (1).

32. System gemäß Anspruch 31, **dadurch gekennzeichnet, dass** es zusätzlich zu der Durchstoßvorrichtung eine Perforationsvorrichtung in Form einer Lanzette, einer Kanüle, einer Nadel, eines Messers oder eines Dorns enthält, zum Erzeugen einer Perforation in der Haut eines Patienten, um eine Körperflüssigkeit als flüssige Probe (20) zu entnehmen, oder dass die Durchstoßvorrichtung auch als Perforationsvorrichtung dient.

33. System gemäß Anspruch 32, **dadurch gekennzeichnet, dass** die Perforationsvorrichtung von der Durchstoßvorrichtung konzentrisch umgeben ist.

34. System gemäß Anspruch 32, **dadurch gekennzeichnet, dass** die Durchstoßvorrichtung und die Perforationsvorrichtung in einer Halterung nebeneinander angeordnet sind und durch eine Drehung der Halterung nacheinander in eine Arbeitsposition bringbar sind.

35. System gemäß einem der Ansprüche 31 bis 34, **gekennzeichnet durch** ein Testelement-Magazin, das ein Band (23) aus aneinandergereihten Testelementen (1) ist, wobei das System eine Transporteinrichtung umfasst, die so ausgebildet ist, dass sie nacheinander jeweils ein Testelement (1) in dem Band (23) in eine Arbeitsposition transportieren kann, in der die Versiegelung des Testelements (1) **durch** die Durchstoßvorrichtung geöffnet werden kann.

36. System gemäß einem der Ansprüche 31 bis 34, **gekennzeichnet durch** ein Testelement-Magazin, das eine Diskette (28) ist, auf der die Testelemente (1) radial angeordnet sind, wobei jeweils ein Testelement (1) **durch** Drehung der Diskette (28) in eine Arbeitsposition drehbar ist, in der die Versiegelung des Testelements (1) **durch** die Durchstoßvorrichtung geöffnet werden kann.

37. System gemäß einem der Ansprüche 31 bis 36, umfassend eine Kompressionseinheit (24) zur Erhöhung des Drucks auf perforierte Haut des Patienten bei der Entnahme der Körperflüssigkeit.

38. Verwendung eines analytischen Testelements gemäß einem der Ansprüche 1 bis 29 zur Analyse des Glukosegehaltes im Blut oder in interstitieller Flüssigkeit.

## Claims

1. Analytical test element for analysing a fluid sample (20), containing a suitable channel (9) for capillary transport of the fluid sample (20), with an entry opening (7) for the fluid sample (20), and a venting opening (8), at least one test panel (17) being arranged in the channel (9) at a distance from the entry opening (7), and the test element (1) including a sample receiving position (4) which is closed by a seal, said sample receiving position (4) being designed so that through an opening in the seal the sample receiving position (4) and the entry opening (7) of the channel (9) can be opened simultaneously to the environment of the test element (1), and so that the fluid sample (20) can be received by the test element (1) via the sample receiving position (4) and the entry opening (7) into the channel (9), for analysis in the test panel (17), it being possible to open the sample receiving position (4), the entry opening (7) of the channel (9) and the venting opening (8) of the channel (9) simultaneously to the environment of the test element (1) by opening the seal by puncturing or cutting open, **characterized in that** the entry opening (7) and the venting opening (8) of the channel (9) open into a clear space (6) in the test element (1), said clear space (6) being arranged so that it is opened to the environment of the test element (1) when the seal is opened.

2. Analytical test element according to Claim 1, **characterized in that** the entry opening (7) is arranged at one end of the channel (9), and the venting opening (8) is arranged at the other end of the channel (9).

3. Analytical test element according to Claim 1, **characterized in that** in the unused state of the test element (1), the free space (6), on a first side, is delimited at least partly by the seal which transposes the sample receiving position (4), and on a second side opposite the first side, is delimited at least partly by a second seal.

4. Analytical test element according to one of Claims 1 to 3, **characterized in that** the channel (9) in the test element (1) runs in a u shape or v shape.

5. Analytical test element according to one of Claims 1 to 4, **characterized in that** the channel (9) has an essentially rectangular cross-section, a width of < 5 mm and a height of < 1 mm.

6. Analytical test element according to one of Claims 1 to 5, **characterized in that** the channel (9) has a total length of < 5 cm.

7. Analytical test element according to one of Claims 1 to 6, **characterized in that** the distance between the sample receiving position (4) and the entry opening (7) of the channel (9) is less than the distance between the sample receiving position (4) and the venting opening (8) of the channel (9).

8. Analytical test element according to one of Claims 1. to 7, **characterized in that** the channel (9) has better wettability by the fluid sample in the region of the entry opening (7) than in the region of the venting opening (8).

9. Analytical test element according to one of Claims 1 to 8, **characterized in that** the channel (9) internally has better wettability by the fluid sample (20) between the entry opening (7) and the at least one test panel (17) than between the at least one test panel (17) and the venting opening (8).

10. Analytical test element according to one of Claims 1 to 9, **characterized in that** the seal which closes the sample receiving position (4) has better wettability by the fluid sample (20) on the side facing the inside of the test element (1) than on the side facing away from the inside of the test element (1).

11. Analytical test element according to one of Claims 1 to 10, **characterized in that** a drying agent (19) is included in the test element (1).

12. Analytical test element according to Claim 11, **characterized in that** the drying agent (19) contains zeoliths or silica gel.

13. Analytical test element according to one of Claims 1 to 12, **characterized by** a waste region (21) to receive an excess (22) of fluid sample (20) in the test element (1).

14. Analytical test element according to one of Claims 1 to 13, **characterized in that** the seal which closes the sample receiving position (4) has prestructuring in such a form that when the seal is opened, a defined burr occurs.

15. Analytical test element according to Claim 14, **characterized in that** the prestructuring is cross-shaped or star-shaped.

16. Analytical test element according to one of Claims 1 to 15, **characterized in that** the test element (1) includes a detection window (18) facing the test panel (17), for photometric analysis of the fluid sample (20) on the test panel (17).

17. Analytical test element according to one of Claims 1 to 16, **characterized in that** in at least one wall delimiting the channel (9), tracks for electrochemical analysis of the fluid sample (20) are placed on the test panel (17).

18. Analytical test element according to one of Claims 1 to 17, **characterized in that** the test element (1) is built up of multiple layers.

19. Analytical test element according to Claim 18, **characterized in that** the channel (9) runs through one or more layers.

20. Analytical test element according to one of Claims 18 or 19, **characterized in that** the test element (1) includes at least one sealing layer (2, 3).

21. Analytical test element according to Claim 20, **characterized in that** the sealing layer (2, 3) has a hydrophobic coating (5).

22. Analytical test element according to one of Claims 18 to 21, **characterized in that** the test element (1) includes at least one carrier layer (12, 13, 14) and at least one intermediate layer (10, 11) which at least partly contains the channel (9).

23. Analytical test element according to one of Claims 18 to 22, **characterized in that** the layers are arranged in the following sequence:
A) a first sealing layer (2) which surrounds the sample receiving position (4),
B) a transparent first carrier layer (13),
C) a first intermediate layer (10) which contains a first part (30) of the channel (9),
D) a second carrier layer (12) which contains an opening (32),
E) a second intermediate layer (11) which contains a second part (31) of the channel (9),
F) a third carrier layer (14), and
G) a second sealing layer (3), the opening (32) in the second carrier layer (12) joining the first part (30) of the channel (9) to the second part (31) of the channel (9), and a test panel (17) being arranged in the first part of the channel (9), and if the test element (1) is unused, a clear space (6), into which the first and second parts (30, 31) of the channel (9) open, extending through the layers B) to F).

24. Analytical test element according to Claim 23, **characterized in that** the first sealing layer (2) on its outside, and the sides of the second and third carrier layers (12, 14) facing the second part (31) of the channel (9), have a hydrophobic coating (15, 16).

25. Analytical test element according to Claim 23 or 24, **characterized in that** in the second part (31) of the channel (9), a drying agent (19) is arranged.

26. Analytical test element according to one of Claims 23 to 25, **characterized in that** the first sealing layer (2) includes a detection window (18) for photometric analysis of the fluid sample (20) on the test panel (17).

27. Analytical test element according to one of Claims 23 to 26, **characterized in that** the intermediate layers (10, 11) are hydrophobic.

28. Analytical test element according to one of Claims 23 to 27, **characterized in that** the sides of the first and second carrier layers (13, 12) facing the first part (30) of the channel (9) are hydrophilic or have a hydrophilic coating.

29. Analytical test element according to one of Claims 1 to 28, **characterized in that** the seal or sealing layer (2, 3) is a compound foil, which includes an aluminium foil, an outer layer of PE, PET or oriented PA, and an inner layer of PE, PP or enamels, or which includes a high barrier compound of PET/SiOₓ.

30. Test element magazine with multiple test elements (1) according to one of Claims 1 to 29, the test element magazine being in the form of a ribbon (23), drum, stack or diskette (28).

31. System for analysing fluid samples (20), with at least one analytical test element (1) according to one of Claims 1 to 29, and a puncturing device to open the seal of one test element (1) at a time at the sample receiving position (4) shortly before receiving a fluid sample (20), and with a detector (25) to analyse the fluid samples (20) in the test panel (17) of a test element (1).

32. System according to Claim 31, **characterized in that** in addition to the puncturing device, it includes a perforation device in the form of a lancet, a cannula, a needle, a knife or a mandrel, to generate a perforation in the skin of a patient, to take a body fluid as a fluid sample (20), or that the puncturing device is also used as a perforation device.

33. System according to Claim 32, **characterized in that** the perforation device is concentrically surrounded by the puncturing device.

34. System according to Claim 32, **characterized in that** the puncturing device and the perforation device are arranged next to each other in a holder, and can be brought into a working position one after another by rotating the holder.

35. System according to one of Claims 31 to 34, **characterized by** a test element magazine which is a ribbon (23) of test elements (1) strung together, the system including a transport device which is in such a form that it can transport one test element (1) in the ribbon (23) after another into a working position, in which the seal of the test element (1) can be opened by the puncturing device.

36. System according to one of Claims 31 to 34, **characterized by** a test element magazine which is a diskette (28), on which the test elements (1) are arranged radially, it being possible, by rotating the diskette (28), to rotate one test element (1) at a time into a working position, in which the seal of the test element (1) can be opened by the puncturing device.

37. System according to one of Claims 31 to 36, including a compression unit (24) to raise the pressure on the patient's perforated skin when the body fluid is removed.

38. Use of an analytical test element according to one of Claims 1 to 29 to analyse the glucose content in blood or interstitial fluid.

## Revendications

1. Elément de test analytique pour l'analyse d'un échantillon (20) liquide contenant un canal (9) approprié pour le transport capillaire de l'échantillon (20) liquide avec une ouverture d'entrée (7) pour l'échantillon (20) liquide et une ouverture de ventilation (8), au moins une zone de test (17) étant disposée dans le canal (9) à distance de l'ouverture d'entrée (7) et l'élément de test (1) comprenant un emplacement de réception d'échantillon (4), fermé avec un scellement, qui est conçu de telle sorte qu'en même temps l'emplacement de réception d'échantillon (4) et l'ouverture d'entrée (7) du canal (9) peuvent être ouverts en direction de l'environnement de l'élément de test (1) par une ouverture du scellement et l'échantillon (20) liquide traversant l'élément de test (1), passant par l'emplacement de réception d'échantillon (4) et l'ouverture d'entrée (7) et entrant dans le canal (9) peut être réceptionnée dans la zone de test (17) pour l'analyse, l'emplacement de réception d'échantillon (4), l'ouverture d'entrée (7) du canal (9) et l'ouverture de ventilation (8) du canal (9) pouvant être ouverts simultanément en direction de l'environnement de l'élément de test (1) par une ouverture du scellement par percée ou ouverture en coupant, **caractérisé en ce que** l'ouverture d'entrée (7) et l'ouverture de ventilation (8) du canal (9) débouchent dans un espace libre (6) dans l'élément de test (1), qui est disposé de telle sorte qu'il est ouvert lors de l'ouverture du scellement en direction de l'environnement de l'élément de test (1).

2. Elément de test analytique selon la revendication 1, **caractérisé en ce que** l'ouverture d'entrée (7) est disposée sur une extrémité du canal (9) et l'ouverture de ventilation (8) sur l'autre extrémité du canal (9).

3. Elément de test analytique selon la revendication 1, **caractérisé en ce que** l'espace libre (6) est limité, lorsque l'élément de test (1) n'est pas utilisé, sur un premier côté au moins en partie par le scellement fermant l'emplacement de réception d'échantillon (4) et sur un second côté opposé au premier côté au moins en partie par un second scellement.

4. Elément de test analytique selon l'une quelconque des revendications 1 à 3, **caractérisé en ce que** le canal (9) est en forme de u ou de v dans l'élément de test (1).

5. Elément de test analytique selon l'une quelconque des revendications 1 à 4, **caractérisé en ce que** le canal (9) présente une section sensiblement rectangulaire et une largeur < 5 mm et une hauteur < 1 mm.

6. Elément de test analytique selon l'une quelconque des revendications 1 à 5, **caractérisé en ce que** le canal (9) présente une longueur totale < 5 cm.

7. Elément de test analytique selon l'une quelconque des revendications 1 à 6, **caractérisé en ce que** la distance de l'emplacement de réception d'échantillon (4) à l'ouverture d'entrée (7) du canal (9) est inférieure à la distance de l'emplacement de réception d'échantillon (4) à l'ouverture de ventilation (8) du canal (9).

8. Elément de test analytique selon l'une quelconque des revendications 1 à 7, **caractérisé en ce que** le canal (9) présente dans la zone de l'ouverture d'entrée (7) une meilleure mouillabilité du fait de l'échantillon liquide que dans la zone de l'ouverture de ventilation (8).

9. Elément de test analytique selon l'une quelconque des revendications 1 à 8, **caractérisé en ce que** le canal (9) présente dans son intérieur entre l'ouverture d'entrée (7) et la au moins une zone de test (17) une meilleure mouillabilité du fait de l'échantillon (20) liquide que entre la au moins une zone de test (17) et l'ouverture de ventilation (8).

10. Elément de test analytique selon l'une quelconque des revendications 1 à 9, **caractérisé en ce que** le scellement fermant l'emplacement de réception d'échantillon (4) présente sur le côté tourné vers le côté intérieur de l'élément de test (1) une meilleure mouillabilité du fait de l'échantillon (20) liquide que sur le côté opposé au côté intérieur de l'élément de test (1).

11. Elément de test analytique selon l'une quelconque des revendications 1 à 10, **caractérisé en ce qu'**un agent déshydratant (19) est inclus dans l'élément de test (1).

12. Elément de test analytique selon la revendication 11, **caractérisé en ce que** l'agent déshydratant (19) contient des zéolites ou du silicagel.

13. Elément de test analytique selon l'une quelconque des revendications 1 à 12, **caractérisé par** une zone de rejet (21) pour la réception d'un excédent (22) d'échantillon (20) liquide dans l'élément de test (1).

14. Elément de test analytique selon l'une quelconque des revendications 1 à 13, **caractérisé en ce que** le scellement fermant l'emplacement de réception d'échantillon (4) présente une pré-structuration qui est conçue de telle sorte qu'une nervure définie se forme lors de l'ouverture du scellement.

15. Elément de test analytique selon la revendication 14, **caractérisé en ce que** la pré-structuration est en forme de croix ou d'étoile.

16. Elément de test analytique selon l'une quelconque des revendications 1 à 15, **caractérisé en ce que** l'élément de test (1) contient une fenêtre de détection (18) tournée vers la zone de test (17) pour l'analyse photométrique de l'échantillon (20) liquide sur la zone de test (17).

17. Elément de test analytique selon l'une quelconque des revendications 1 à 16, **caractérisé en ce que** des pistes conductrices sont introduites dans au moins une paroi délimitant le canal (9) pour une analyse électrochimique de l'échantillon (20) liquide sur la zone de test (17).

18. Elément de test analytique selon l'une quelconque des revendications 1 à 17, **caractérisé en ce que** l'élément de test (1) est conçu à base d'une pluralité de couches.

19. Elément de test analytique selon la revendication 18, **caractérisé en ce que** le canal (9) passe par une ou plusieurs couches.

20. Elément de test analytique selon l'une quelconque des revendications 18 ou 19, **caractérisé en ce que** l'élément de test (1) comprend au moins une couche de scellement (2, 3).

21. Elément de test analytique selon la revendication 20, **caractérisé en ce que** la couche de scellement (2, 3) présente un revêtement (5) hydrophobe.

22. Elément de test analytique selon l'une quelconque des revendications 18 à 21, **caractérisé en ce que** l'élément de test (1) comporte au moins une couche support (12, 13, 14) et au moins une couche intermédiaire (10, 11) contenant au moins en partie le canal (9).

23. Elément de test analytique selon l'une quelconque des revendications 18 à 22, **caractérisé en ce que** les couches sont disposées dans l'ordre de succession suivant :
A) une première couche de scellement (2) comprenant un emplacement de réception d'échantillon (4),
B) une première couche support (13) transparente,
C) une première couche intermédiaire (10) contenant une première partie (30) du canal (9),
D) une seconde couche support (12) contenant une ouverture (32),
E) une seconde couche intermédiaire (11) contenant une seconde partie (31) du canal (9),
F) une troisième couche support (14) et
G) une seconde couche de scellement (3),
l'ouverture (32) dans la seconde couche support (12) reliant la première partie (30) du canal (9) à la seconde partie (31) du canal (9) et une zone de test (17) étant disposée dans la première partie du canal (9), et un espace libre (6) s'étendant à travers les couches B) jusqu'à F) sur l'élément de test (1) non utilisé, espace dans lequel la première et la seconde parties (30, 31) du canal (9) débouchent.

24. Elément de test analytique selon la revendication 23, **caractérisé en ce que** la première couche de scellement (2) sur son côté extérieur et les côtés, tournées vers la seconde partie (31) du canal (9), de la seconde et de la troisième couches supports (12, 14) présentent un revêtement (15, 16) hydrophobe.

25. Elément de test analytique selon l'une quelconque des revendications 23 ou 24, **caractérisé en ce qu'**un agent déshydratant (19) est disposé dans la seconde partie (31) du canal (9).

26. Elément de test analytique selon l'une quelconque des revendications 23 à 25, **caractérisé en ce que** la première couche de scellement (2) contient une fenêtre de détection (18) pour l'analyse photométrique de l'échantillon (20) liquide sur la zone de test (17).

27. Elément de test analytique selon l'une quelconque des revendications 23 à 26, **caractérisé en ce que** les couches intermédiaires (10, 11) sont hydrophobes.

28. Elément de test analytique selon l'une quelconque des revendications 23 à 27, **caractérisé en ce que** les côtés, tournés vers la première partie (30) du canal (9), de la première et de la seconde couches supports (13, 12), sont hydrophiles ou revêtus de façon hydrophile.

29. Elément de test analytique selon l'une quelconque des revendications 1 à 28, **caractérisé en ce que** le scellement ou la couche de scellement (2, 3) est un film composite qui comporte une feuille d'alu, une couche extérieure en PE, PET ou PA orienté et une couche intérieure à base de PE, PP ou de laques qui comporte un composite à barrière élevée à base de PET/SiOₓ.

30. Magasin d'élément de test comprenant une pluralité d'éléments de test (1) selon l'une quelconque des revendications 1 à 29, le magasin d'éléments de test étant conçu sous forme de bande (23), de tambour, de pile ou de disquette (28).

31. Système pour l'analyse d'échantillons (20) liquides avec au moins un élément de test (1) analytique selon l'une quelconque des revendications 1 à 29 et un dispositif de percée pour l'ouverture du scellement de respectivement un élément de test (1) sur l'emplacement de réception d'échantillon (4) juste avant la réception d'un échantillon (20) liquide et avec un détecteur (25) pour l'analyse des échantillons (20) liquides dans la zone de test (17) d'un élément de test (1).

32. Système selon la revendication 31, **caractérisé en ce que**, en supplément du dispositif de percée, il contient un dispositif de perforation sous la forme d'une lancette, d'une canule, d'une aiguille, d'un couteau ou d'un aiguillon, pour générer une perforation dans la peau d'un patient, afin de prélever un liquide de corps sous forme d'échantillon (20) liquide, ou **en ce que** le dispositif de percée sert également de dispositif de perforation.

33. Système selon la revendication 32, **caractérisé en ce que** le dispositif de percée est entouré de façon concentrique par le dispositif de percée.

34. Système selon la revendication 32, **caractérisé en ce que** le dispositif de percée et le dispositif de perforation sont disposés l'un à côté de l'autre dans un support et peuvent être amenés par une rotation du support l'un après l'autre dans une position de travail.

35. Système selon l'une quelconque des revendications 31 à 34, **caractérisé par** un magasin d'élément de test, qui est une bande (23) à base d'éléments de test (1) alignés, le système comportant un système de transport qui est conçu de telle sorte qu'il peut transporter successivement à chaque fois un élément de test (1) dans la bande (23) dans une position de travail dans laquelle le scellement de l'élément de test (1) peut être ouvert par le dispositif de perforation.

36. Système selon l'une quelconque des revendications 31 à 34, **caractérisé par** un magasin d'élément de test, qui est une disquette (28), sur laquelle les éléments de test (1) sont disposés radialement, un élément de test (1) pouvant être tourné à chaque fois par rotation de la disquette (28) dans une position de travail dans laquelle le scellement de l'élément de test (1) peut être ouvert par le dispositif de percée.

37. Système selon l'une quelconque des revendications 31 à 36, comprenant une unité de compression (24) pour l'élévation de la pression sur la peau perforée du patient lors du prélèvement du liquide de corps.

38. Utilisation d'un élément de test analytique selon l'une quelconque des revendications 1 à 29 pour l'analyse de la teneur en glucose dans le sang ou dans le liquide interstitiel.
